# DEMANDE DE BREVET EUROPEEN

(11) **EP 4 129 171 A1**
(43) Date de publication de la demande: **08.02.2023**
(21) Numéro de dépôt: 22185365.8
(22) Date de dépôt: 18.07.2022
(51) Int. Cl.: A61B 5/1172, G06V 40/60

(54) **DISPOSITIF DE CAPTURE D'EMPREINTE BIOMETRIQUE ERGONOMIQUE**

(30) Priorité: 05.08.2021 FR 2108507
(71) Demandeur: Idemia Identity & Security France, 92400 Courbevoie (FR)
(72) Inventeur: FOURRE, Joël-Yann, 92400 Courbevoie (FR); LE GOUIL, Elise, 92400 Courbevoie (FR); MALEK, Mokrane, 92400 Courbevoie (FR)
(74) Mandataire: Idemia

(57) **Abrégé**

L'invention porte sur un dispositif de capture d'empreinte biométrique (1) d'un utilisateur, le compartiment utilisateur (10) comportant une ouverture frontale (11) adaptée pour le passage de la partie d'une main au sein dudit compartiment utilisateur (10) et une ouverture latérale (121, 122) permettant le passage du pouce de la main de l'utilisateur, l'ouverture latérale (121, 122) s'étendant jusqu'à l'ouverture frontale (11) pour former un espace ouvert continu, l'ouverture latérale (121, 122) étant délimitée selon une direction d'insertion (Din) de la main de l'utilisateur dans le compartiment utilisateur (10) par une butée de positionnement (B1, B2) de la main de l'utilisateur.

## Description

L'invention concerne le domaine des dispositifs de capture d'empreinte biométrique d'une partie d'une main telle que la paume de la main ou des doigts de la main, en particulier sans contact.

Il est connu de l'état de la technique des dispositifs de capture d'empreinte biométrique apte à acquérir une image d'une empreinte de paume de main, avec contact. Pour une acquisition d'une image d'une empreinte de paume de bonne qualité, en vue d'une authentification biométrique, il est souhaitable que le pouce de la main soit écarté de l'index de ladite main afin de tendre la peau entre ledit pouce et ledit index, pour éviter la création de plis de la peau. Ainsi, des dispositifs connus affichent un gabarit de main dont le pouce est écarté de l'index, l'utilisateur étant invité à poser sa main sur le gabarit pour l'acquisition de l'image d'empreinte biométrique. La main est ainsi bien positionnée pour l'acquisition d'une image d'empreinte biométrique. Un problème d'une telle solution est que l'utilisateur pose sa main sur une surface du dispositif, l'acquisition n'est pas sans contact avec le dispositif. Pour des questions d'hygiène, il est préférable de privilégier l'utilisation de solutions sans contact pour éviter la contamination par des agents pathogènes des surfaces touchées par des utilisateurs successifs. Un autre problème d'une telle solution est l'inadéquation de la forme de la surface plane du dispositif vis-à-vis de la paume d'une main qui est de forme courbe. Le contact entre la paume et le dispositif n'est garanti sur toute la surface qu'en écrasant la main de l'utilisateur sur la surface dudit dispositif.

Il est connu de l'état de la technique des dispositifs de capture d'empreinte biométrique apte à acquérir une image d'une empreinte de main ou d'une partie d'une main, sans contact. Ces dispositifs n'incitent pas l'utilisateur à écarter de manière intuitive le pouce de l'index, ni à un bon positionnement naturel de la partie de main vis à vis dudit dispositif. Ils nécessitent le guidage de l'utilisateur par un opérateur qualifié pour garantir une acquisition d'une image d'une empreinte de paume de main de bonne qualité.

L'invention vise à résoudre les problèmes susmentionnés de l'état de la technique en proposant une solution ergonomique et intuitive pour l'utilisateur pour une acquisition d'image d'empreinte biométrique de qualité.

L'invention porte sur un dispositif de capture d'empreinte biométrique d'une partie d'une main d'un utilisateur comprenant un compartiment électronique et un compartiment utilisateur, le compartiment utilisateur comportant une ouverture frontale adaptée pour le passage de la partie d'une main au sein dudit compartiment utilisateur, le compartiment électronique comprenant des moyens d'acquisition d'une image d'une empreinte de ladite partie d'une main, le compartiment utilisateur comprenant au moins une ouverture latérale permettant le passage du pouce de la main de l'utilisateur, l'ouverture latérale s'étendant jusqu'à l'ouverture frontale pour former un espace ouvert continu, l'ouverture latérale étant délimitée selon une direction d'insertion de la main de l'utilisateur dans le compartiment utilisateur par une butée de positionnement de la main de l'utilisateur.

Selon un aspect de l'invention, le dispositif de capture d'empreinte biométrique est un dispositif sans contact.

Selon un aspect de l'invention, le compartiment utilisateur comprend une zone d'acquisition délimitée du côté de l'ouverture frontale par une limite, la butée de positionnement étant localisée à une distance à la butée comprise entre cinquante et quatre-vingts millimètres de ladite limite.

Selon un aspect de l'invention, le compartiment utilisateur comprend un orifice de passage apte à laisser dépasser dudit compartiment utilisateur une extrémité de ladite main de l'utilisateur, l'orifice de passage faisant face à l'ouverture frontale.

Selon un aspect de l'invention, le compartiment utilisateur comprend une face supérieure comportant une portion transparente permettant à l'utilisateur de voir au moins une portion de la partie de main pour en ajuster le positionnement au sein dudit compartiment utilisateur.

Selon un aspect de l'invention, le compartiment électronique comprend des moyens d'éclairage aptes à générer au moins un faisceau lumineux en direction du compartiment utilisateur.

Selon un aspect de l'invention, le compartiment utilisateur comprend des moyens de blocage au moins partiel d'un faisceau lumineux provenant du compartiment électronique au niveau de la portion transparente.

Selon un aspect de l'invention, le compartiment utilisateur comprend une visière de blocage apte à bloquer un faisceau lumineux provenant du compartiment électronique, au niveau d'au moins une ouverture parmi l'ouverture latérale et l'ouverture frontale.

Selon un aspect de l'invention, le compartiment utilisateur comprenant une visière de blocage apte à bloquer un faisceau lumineux provenant du compartiment électronique, au niveau d'au moins une ouverture parmi l'ouverture latérale, l'ouverture frontale et l'orifice de passage.

Selon un aspect de l'invention, le compartiment utilisateur comprend une face latérale comportant ladite ouverture latérale s'étendant depuis l'ouverture frontale du compartiment utilisateur jusqu'à la butée de positionnement.

Selon un aspect de l'invention, l'ouverture latérale est délimitée par la butée de positionnement, un bord supérieur et un bord inférieur.

Selon un aspect de l'invention, au moins un des bords parmi le bord supérieur et le bord inférieur est agencé de sorte à définir un plan de positionnement moyen naturel pour la main sensiblement parallèle à l'au moins un des bords.

Selon un aspect de l'invention, au moins un des bords parmi le bord supérieur et le bord inférieur est agencé de sorte à définir un plan de positionnement moyen naturel pour la main, sensiblement équidistant du bord supérieur et du bord inférieur.

Selon un aspect de l'invention, le dispositif de capture d'empreinte biométrique comprend deux ouvertures latérales de part et d'autre de l'ouverture frontale, une première ouverture latérale permettant le passage du pouce de la main droite de l'utilisateur, une deuxième ouverture latérale permettant le passage du pouce de la main gauche de l'utilisateur.

Selon un aspect de l'invention, le dispositif de capture d'empreinte biométrique comprend des moyens pour informer un utilisateur d'une fin de session d'acquisition par les moyens d'acquisition, en fonction d'une distance entre ladite partie de main et la butée de positionnement.

D'autres avantages et caractéristiques de l'invention apparaîtront à la lecture de la description et des dessins.
La figure 1 représente un dispositif de capture d'empreinte biométrique selon l'invention, posé sur une surface plane horizontale.
La figure 2 représente une main d'utilisateur au sein du compartiment utilisateur du dispositif de capture d'empreinte biométrique.
La figure 3 représente un dispositif de capture d'empreinte biométrique selon l'invention, fixé contre un mur.
La figure 4 représente le même dispositif que sur la figure 1, différents éléments constitutifs étant référencés,
La figure 5a représente un premier mode de réalisation du dispositif de capture d'empreinte biométrique selon l'invention.
La figure 5b représente un plan de positionnement naturel pour une main au sein du dispositif de la figure 5a.
La figure 6a illustre une vue de côté d'une première variante d'un premier mode de réalisation.
La figure 6b illustre une vue de côté d'une deuxième variante du premier mode de réalisation.
La figure 6c illustre une vue de côté d'une troisième variante du premier mode de réalisation.
La figure 6d illustre une vue de côté d'une quatrième variante du premier mode de réalisation.
La figure 6e illustre une vue de côté d'une cinquième variante du premier mode de réalisation.
La figure 6f illustre une vue de côté d'une sixième variante du premier mode de réalisation.
La figure 7a illustre une vue de face d'une première variante d'un deuxième mode de réalisation.
La figure 7b illustre une vue de côté de la première variante du deuxième mode de réalisation,
La figure 8a illustre une vue de face d'une deuxième variante du deuxième mode de réalisation.
La figure 8b illustre une vue de côté de la deuxième variante du deuxième mode de réalisation.
La figure 9 représente un dispositif de capture d'empreinte biométrique comprenant trois visières de blocage opaques anti-éblouissement.
La figure 10 représente une main d'utilisateur introduite dans le dispositif de la figure 9, pour une acquisition d'image d'empreinte de paume d'une main.
La figure 11 représente une autre vue de la figure 10.

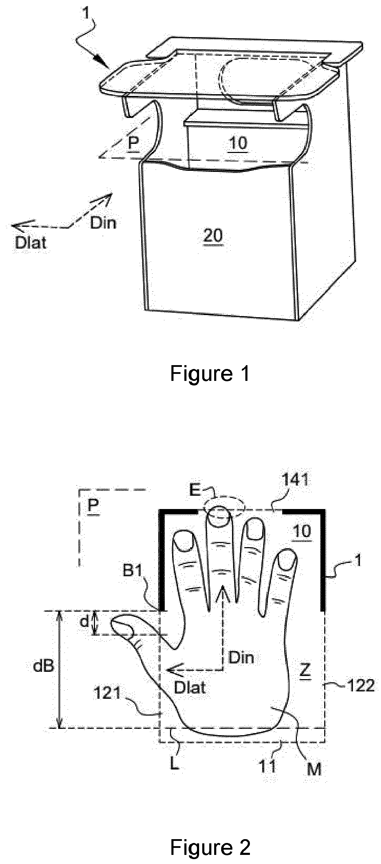

Les figures 1, 3 et 4 représentent un dispositif de capture d'empreinte biométrique 1 selon l'invention.

Le dispositif de capture d'empreinte biométrique 1 est apte à faire l'acquisition d'une image d'une empreinte de partie d'une main d'un utilisateur, par exemple une main, la paume d'une main, un ou plusieurs doigts d'une main.

En particulier, le dispositif est adapté pour acquérir une partie de main étant une partie de paume de main ou une paume de main entière.

Une image d'une empreinte est par exemple une image d'un réseau de crêtes et/ou sillons de la peau, ou d'un réseau veineux présent dans la main.

Le dispositif de capture d'empreinte biométrique 1 est divisé en un compartiment utilisateur 10 et un compartiment électronique 20.

La division du dispositif de capture d'empreinte biométrique 1 en un compartiment utilisateur 10 et un compartiment électronique 20 est une division fonctionnelle.

Par exemple, le dispositif de capture d'empreinte biométrique 1 peut comprendre un seul bloc sensiblement parallélépipédique comportant des parois communes au compartiment utilisateur 10 et au compartiment électronique 20 ou bien être un assemblage d'un bloc physique de compartiment utilisateur et d'un bloc physique de compartiment électronique différent.

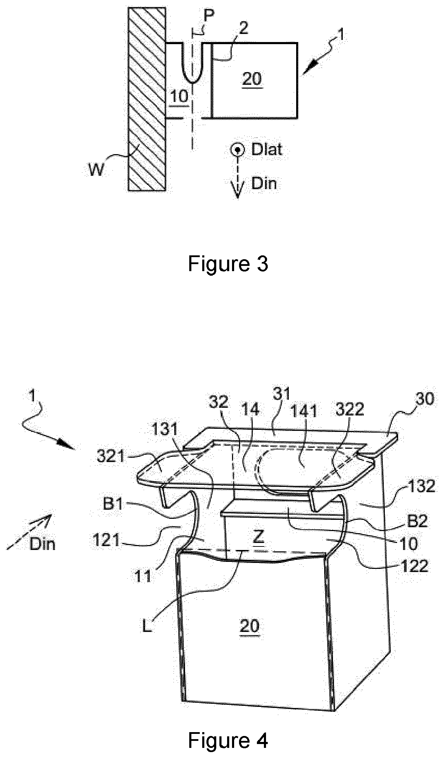

Le compartiment utilisateur 10 et le compartiment électronique 20 peuvent être cloisonnés l'un par rapport à l'autre, par exemple par une vitre de séparation 2 tel que représenté en figure 3. La vitre de séparation peut se trouver à la jonction de deux compartiments physique différents, ou localisée dans un compartiment parmi le compartiment utilisateur 10 et le compartiment électronique 20.

Le compartiment utilisateur 10 et le compartiment électronique 20 peuvent être ouvert l'un sur l'autre tel que représenté en figure 1, sans cloison physique entre les deux.

Le compartiment utilisateur 10 est apte à recevoir en son sein une partie d'une main d'un utilisateur selon un plan de positionnement P moyen naturel pour la main, en vue d'une capture d'empreinte biométrique sans contact par le compartiment électronique 20.

Le dispositif de capture d'empreinte biométrique 1 est sans contact en ce que la capture d'empreinte biométrique est réalisée sans contact de la partie de main avec une surface d'acquisition, contrairement à un dispositif de capture avec contact qui comprend une surface d'acquisition prévue pour recevoir une partie de main en appui, en vue de son acquisition.

La partie de main d'un utilisateur dont l'empreinte biométrique doit être imagée, par exemple une paume de main, doit être tournée vers le compartiment électronique 20.

Le plan de positionnement P moyen naturel pour la main est défini par les deux directions ci-dessous
- une direction d'insertion Din de la main dans le compartiment utilisateur 10,
- une direction latérale Dlat perpendiculaire à la direction d'insertion Din.

La figure 1 illustre un dispositif de capture d'empreinte biométrique 1 posé sur une surface plane tel qu'une table ou le sol. Le plan de positionnement P moyen naturel pour la main est horizontal.

La figure 2 illustre une vue de dessus et en coupe selon le plan de positionnement P, d'une main M disposée au sein du compartiment utilisateur 10 du dispositif de capture d'empreinte biométrique 1 selon la figure 1, en vue d'une capture biométrique de la paume de la main. La main M est positionnée selon un plan de positionnement P. La paume de la main est dirigée vers le compartiment électronique 20. La direction d'insertion Din s'étend selon une direction longitudinale de la main, c'est-à-dire allant du poignet de l'utilisateur au doigt majeur de la main. La direction latérale Dlat s'étend selon une direction latérale de la main, c'est-à-dire allant de l'auriculaire au pouce, selon une direction perpendiculaire à la direction d'insertion Din.

Selon la figure 3, le dispositif de capture d'empreinte biométrique 1 est fixé à un mur W. Le plan de positionnement P moyen naturel pour la main est vertical.

Comme visible sur les figures 2 et 4, le compartiment utilisateur 10 comporte une ouverture frontale 11 adaptée pour le passage de la partie de la main de l'utilisateur.

En particulier, l'ouverture frontale 11 est adaptée pour le passage de la paume d'une main de l'utilisateur.

La largeur de l'ouverture frontale 11 selon la direction latérale Dlat, doit permettre l'insertion au sein du compartiment de toutes les tailles de main, en particulier de toutes les tailles de paumes de main, mais ne doit pas être trop grande pour inciter l'utilisateur à insérer sa main selon le plan de positionnement P prédéterminé et aussi à écarter le pouce lors d'une introduction de la paume de la main au sein du compartiment utilisateur 10. Par exemple, la largeur de l'ouverture frontale 11 selon la direction latérale Dlat est comprise entre 100 mm et 150 mm, de préférence entre 120 mm et 130 mm.

Face à l'ouverture frontale 11, le compartiment utilisateur 10 comprend de manière optionnelle et avantageuse un orifice de passage 141.

L'orifice de passage 141 est apte recevoir une extrémité E de ladite main M de l'utilisateur de manière pour laisser dépasser ladite extrémité E du compartiment utilisateur 10, comme l'illustre la figure 2.

La profondeur du compartiment utilisateur 10, entre l'ouverture frontale 11 et l'orifice de passage 141, selon la direction d'insertion Din peut être de 50 mm. Dans ce cas, l'orifice de passage 141 est apte à laisser passer les doigts index, majeur, annulaire et auriculaire d'une main. Le dispositif est alors apte à acquérir une image d'une empreinte d'une partie de la paume d'une main, plusieurs acquisitions en différentes positions selon la direction Din étant alors nécessaires pour pouvoir reconstituer une image d'empreinte de la paume entière de la main ou de la main entière incluant les doigts.

De préférence, la profondeur du compartiment utilisateur 10, entre l'ouverture frontale 11 et l'orifice de passage 141, selon la direction latérale Din est comprise entre 120 mm et 200 mm. En fonction de la taille de la main de l'utilisateur et de la profondeur du compartiment utilisateur 10, l'orifice de passage 141 permet un dépassement d'une extrémité E de la main M de l'utilisateur.

Grâce à l'orifice de passage 141, l'utilisateur n'est pas limité dans son mouvement selon la direction d'insertion Din de la main par un obstacle ne lui permettant pas de bien positionner sa main dans le compartiment utilisateur 10 pour l'acquisition de l'image d'empreinte de la partie de main à imager, en particulier ici, la paume de la main.

L'orifice de passage 141 n'est pas toujours nécessaire, par exemple dans le cas d'une profondeur du compartiment utilisateur 10 de l'ordre de 280 mm.

La hauteur de l'ouverture frontale 11 selon une direction perpendiculaire la direction d'insertion Din et à la direction latérale Dlat, doit permettre le passage d'une main, par exemple comprise entre 50 mm et 100 mm, de préférence entre 60 mm et 80 mm.

Selon un exemple de réalisation, le compartiment utilisateur 10 comprend une paroi de fond 14 faisant face à l'ouverture frontale 11, la paroi de fond 14 comportant l'orifice de passage 141.

Avantageusement, l'orifice de passage 141 est centré sur la paroi de fond 14 selon une direction la latérale Dlat. Cela permet indifféremment le passage d'une extrémité E de la main droite d'un utilisateur ou d'une extrémité E de la main gauche d'un utilisateur

Avantageusement, l'orifice de passage 141 est un repère visuel pour l'utilisateur pour un bon positionnement de la main, l'utilisateur pouvant utiliser l'orifice de passage 141 comme cible de visée pour ledit positionnement de la main.

Avantageusement, l'orifice de passage 141 est un guide en hauteur pour la main de l'utilisateur, c'est à dire selon une direction perpendiculaire à la paume de la main, l'utilisateur pouvant utiliser l'orifice de passage 141 comme guide pour la détermination du plan de positionnement P de la main. De manière préférentielle et tel que représenté figure 4, l'orifice de passage 141 est de forme oblongue, offrant des contours non agressifs pour le passage de l'extrémité E de ladite main de l'utilisateur.

Selon un autre exemple de réalisation, le compartiment utilisateur 10 ne comprend pas de paroi de fond, le compartiment utilisateur étant totalement ouvert sur la partie faisant face à l'ouverture frontale 11.

Le compartiment utilisateur 10 comprend une face supérieure 30.

Avantageusement, la face supérieure 30 comporte une portion transparente 32 permettant à l'utilisateur d'ajuster le positionnement de sa main ou partie de main au sein dudit compartiment utilisateur 10.

La face supérieure 30 fait face au compartiment électronique 20.

La face supérieure 30 peut être parallèle au plan de positionnement P moyen naturel pour la main ou bien former un angle avec celui-ci, par exemple un angle compris en 0 et 45 degrés. Un angle non nul entre la face supérieure 30 et le plan de positionnement P pour la main peut permettre d'améliorer l'ergonomie du dispositif, par exemple faciliter l'insertion de la main de l'utilisateur au sein du compartiment utilisateur 10. Un autre avantage à une inclinaison de la face supérieure 30 par rapport au plan de positionnement P est de limiter de possibles reflets de lumière lorsque la face supérieure 30 comporte une portion transparente 32.

Comme visible sur les figures 2 et 4, le compartiment utilisateur 10 comprend au moins une ouverture latérale 121, 122 permettant le passage du pouce de la main M de l'utilisateur. L'ouverture latérale 121, 122 s'étend jusqu'à l'ouverture frontale 11 pour former un espace ouvert continu.

Avantageusement, l'ouverture frontale 11 s'étend jusqu'à la face supérieure 30.

De manière avantageuse, le compartiment utilisateur 10 comprend deux ouvertures latérales 121, 122 de part et d'autre de l'ouverture frontale 11, une première ouverture latérale 121 permettant le passage du pouce de la main droite de l'utilisateur, une deuxième ouverture latérale 122 permettant le passage du pouce de la main gauche de l'utilisateur.

Une ouverture latérale 121, 122 est délimitée selon la direction d'insertion Din de la main de l'utilisateur, par une butée de positionnement B1, B2 de la main de l'utilisateur.

Une ouverture latérale 121, 122 en coopération avec sa butée de positionnement B1, B2 associée, permet d'inciter l'utilisateur à écarter le pouce de sa main par rapport à l'index de ladite main pour éviter la création de plis sur la paume de la main.

La butée de positionnement gêne une introduction complète de la main, incitant l'utilisateur à écarter le pouce et permet en outre de bloquer une progression de la main au sein du compartiment utilisateur, selon la direction d'insertion Din, lorsque le pouce arrive en butée sur la butée de positionnement.

Le compartiment utilisateur 10 comprend une zone d'acquisition Z délimitée du côté de l'ouverture frontale 11 par une limite L.

La butée de positionnement B1, B2 est avantageusement localisée à une distance à la butée dB comprise entre cinquante et quatre-vingts millimètres de ladite limite L, de préférence entre soixante et soixante-dix millimètres de ladite limite L, la distance à la butée dB étant mesurée dans un plan coplanaire au plan de positionnement P. Un tel positionnement de la butée de positionnement B1, B2 permet l'introduction de la paume d'une main, pouce écarté, au sein du compartiment utilisateur 10 au moins jusqu'à la limite L pour un bon positionnement en vue de la capture de son empreinte biométrique.

Selon un exemple de procédé pour une capture d'empreinte biométrique d'une paume de main, l'utilisateur introduit sa main dans le compartiment utilisateur 10, selon la direction Din d'insertion, par l'ouverture frontale 11. Gêné par la présence de la butée de positionnement B1, B2, l'utilisateur est incité à écarter le pouce de l'index avant même d'entrer en contact avec ladite butée de positionnement B1, B2. Lorsque la ou les acquisitions d'images par les moyens d'acquisition sont terminées, le dispositif 1 signale à l'utilisateur une fin de session d'acquisition. Ainsi, l'utilisateur est informé que sa main et a été suffisamment insérée au sein du compartiment utilisateur 10 pour la capture d'empreinte, et qu'il peut la retirer du compartiment utilisateur 10.

Une fin d'acquisition correspond par exemple au franchissement de la paume de la main de l'utilisateur de la limite L de la zone d'acquisition Z.

Avantageusement, une fin de session d'acquisition est déterminée lorsque la partie de main est à une distance de la butée de positionnement B1, B2, égale à une distance d prédéterminée de l'ordre d'un à deux centimètres.

Une capture d'empreinte peut résulter d'une ou de plusieurs acquisitions d'image par les moyens d'acquisition.

De manière avantageuse, à la fin d'une session d'acquisition, la ou les images prises par moyens d'acquisition sont suffisantes pour générer une image d'empreinte biométrique de la partie de main de l'utilisateur.

Cependant, des images complémentaires peuvent être acquises par moyens d'acquisition pendant le retrait de la main du compartiment utilisateur 10, si nécessaire.

Suivant le positionnement avantageux de la butée de positionnement décrit ci-avant, un bon positionnement pour la capture d'empreinte est atteint avant tout contact de la butée de positionnement B1, B2 avec la partie de main, à une distance de la butée de positionnement B1, B2, supérieure à la distance d prédéterminée de l'ordre d'un à deux centimètres, garantissant ainsi une capture d'empreinte sans contact. Ce positionnement est représenté sur la figure 2.

Le signalement à l'utilisateur est effectué à la distance d prédéterminée de la butée de positionnement B1, B2 de l'ordre d'un à deux centimètres.

Si malgré le signalement de fin de session d'acquisition, l'utilisateur continue d'introduire la paume de sa main dans le compartiment utilisateur 10 selon la direction d'insertion Din, la butée de positionnement B1, B2, arrêtera la progression de la main dans une position dans laquelle la partie de main en contact avec le dispositif reste très localisée et le contact avec le dispositif limité à la butée de positionnement B1, B2.

L'utilisation du dispositif de l'invention est plus hygiénique que les dispositifs avec contact connus de l'état de la technique, même lorsque l'utilisateur ne retire pas sa main après le signalement dudit dispositif.

La direction Din d'insertion est intuitive pour l'utilisateur de par la forme du compartiment utilisateur 10, l'ouverture la plus large étant l'ouverture frontale, les ouvertures latérales 121, 122 étant intuitivement dédiées au passage du pouce de la main.

Néanmoins, des indications ou instructions indiquant à l'utilisateur la direction Din d'insertion peuvent être envisagées, via par exemple un indicateur directionnel, une instruction vocale, une représentation d'un gabarit de main au sein du compartiment utilisateur 10, un pictogramme, ou encore une vidéo affichée sur un écran.

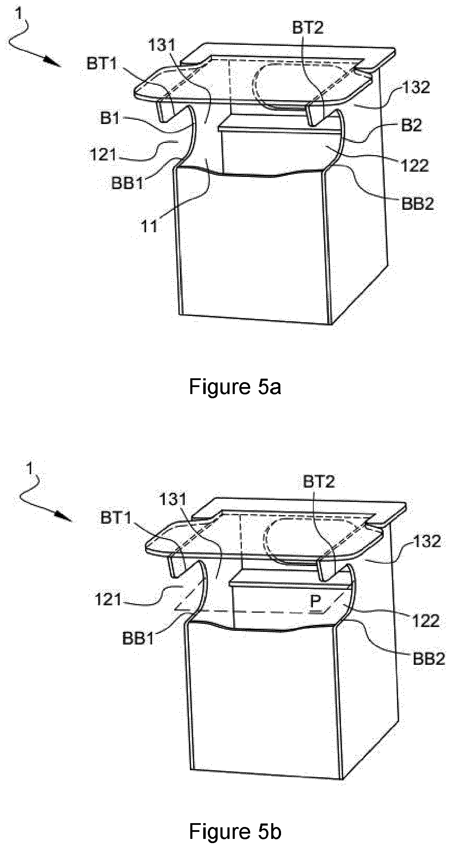

Les figures 5a à 6f illustrent un premier mode de réalisation d'ouverture latérale 121, 122.

Le compartiment utilisateur 10 comprend deux faces latérales 131, 132 comportant chacune une ouverture latérale 121, 122 s'étendant depuis l'ouverture frontale 11 du compartiment utilisateur jusqu'à la butée de positionnement B1, B2.

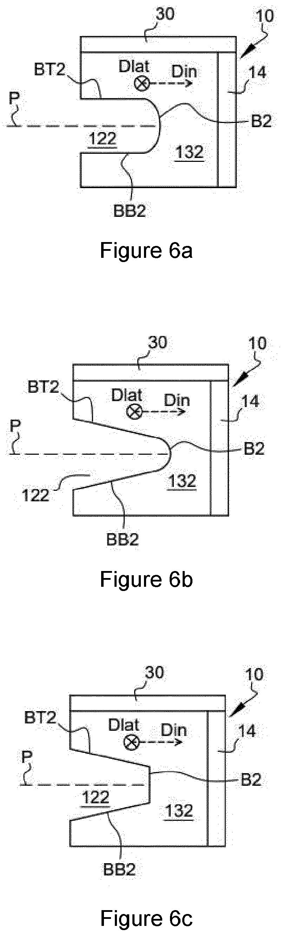

La forme de la butée de positionnement est définie ci-après vu selon un angle de vue depuis la face latérale 132, selon la direction latérale Dlat, tel que représenté sur les figures 6a à 6f, 7b, 8b.

Chaque ouverture latérale 121, 122 est délimitée par la butée de positionnement B1, B2, un bord supérieur BT1, BT2 et un bord inférieur BB1, BB2.

Avantageusement, pour une ouverture latérale 121, 122, au moins un des bords BT1, BT2, BB1, BB2 parmi le bord supérieur BT1, BT2 et le bord inférieur BB1, BB2 est agencé de sorte à définir un plan de positionnement P moyen naturel pour la main sensiblement parallèle à l'au moins un des bords ou équidistant du bord supérieur BT1, BT2 et du bord inférieur BB1, BB2.

Les figures 6a à 6f représentant le compartiment utilisateur 10 d'un dispositif de capture d'empreinte biométrique 1 posé sur un sol horizontal tel que sur la figure 1, vu depuis la face latérale 132, selon la direction latérale Dlat.

Selon une première variante illustrée en figure 6a, les bords supérieur BT2 et inférieur BB2 sont horizontaux. Le plan de positionnement P moyen naturel pour la main est horizontal et parallèle aux bord supérieur BT2 et inférieur BB2, mais aussi à équidistance des bords supérieur BT2 et inférieur BB2. La butée de positionnement B2 est de forme arrondie, en forme de U.

Selon une deuxième variante illustrée en figure 6b, le bord supérieur BT2 est incliné selon un angle négatif dans le sens trigonométrique par rapport à un plan horizontal, le bord inférieur BB2 est incliné selon un angle positif dans le sens trigonométrique par rapport à un plan horizontal, les angles positif et négatif étant opposés et sensiblement de même valeur absolue, par exemple une dizaine de degrés. Le plan de positionnement P moyen naturel est horizontal et est localisé à équidistance des bords supérieur BT2 et inférieur BB2. La butée de positionnement B2 est de forme arrondie, en forme de U.

Une troisième variante est illustrée en figure 6c et diffère de la deuxième variante en ce que la butée de positionnement B2 n'est pas de forme arrondie.

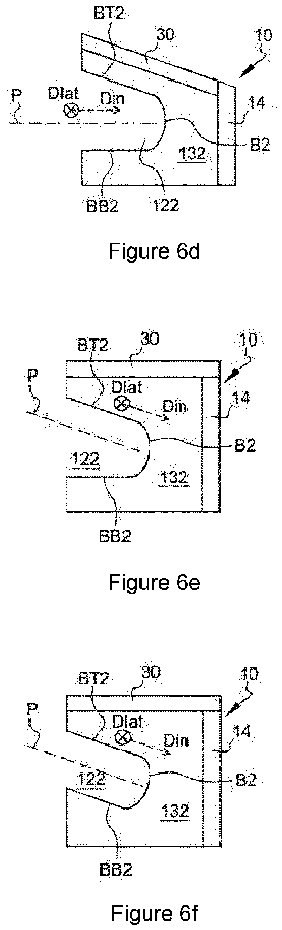

Selon une quatrième variante illustrée en figure 6d, le bord supérieur BT2 est incliné selon un angle négatif par exemple d'une dizaine de degrés, dans le sens trigonométrique par rapport à un plan horizontal, le bord inférieur BB2 est horizontal. Le plan de positionnement P moyen naturel pour la main est horizontal et parallèle au bord inférieur BB2. La butée de positionnement B2 est de forme arrondie, en forme de U.

Selon une cinquième variante illustrée en figure 6e, le bord supérieur BT2 est incliné selon un angle négatif dans le sens trigonométrique par rapport à un plan horizontal, le bord inférieur BB2 est horizontal. Le plan de positionnement P moyen naturel pour la main est incliné et parallèle au bord supérieur BT2. La butée de positionnement B2 est de forme arrondie, en forme de U.

La forme générale du compartiment utilisateur 10 ou des indications par des lignes ou des dessins peuvent favoriser un placement de la main selon un plan de positionnement moyen P prédéterminé.

Par exemple, une couleur différente le long d'un bord de l'orifice latéral 122 peut favoriser un placement de la main selon un plan de positionnement moyen P parallèle à ce bord coloré de manière particulière.

La forme du compartiment peut également inciter à un placement de la main selon un plan de positionnement moyen P particulier. Par exemple, selon les figures 6d et 6e, le plan de positionnement naturel P est différent en fonction de l'inclinaison de la face supérieure 30, l'ouverture latérale 122 ayant la même forme dans les deux cas.

Selon une sixième variante illustrée en figure 6f, le bord supérieur BT2 et le bord inférieur sont inclinés selon un même angle négatif par exemple d'une dizaine de degrés, dans le sens trigonométrique par rapport à un plan horizontal. Le plan de positionnement P moyen naturel est incliné, parallèle aux deux bords supérieur BT2 et inférieur BB2 et est localisé à équidistance des bords supérieur BT2 et inférieur BB2. La butée de positionnement B2 est de forme arrondie, en forme de U.

De manière générale, une forme arrondie pour la butée de positionnement B1, B2 est préférée, quelle que soit l'agencement du bord supérieur BT1, BT2 et du bord inférieur BB1, BB2, pour des raisons d'ergonomie.

Ces six variantes illustrent des exemples non limitatifs de forme possible pour les ouvertures latérales 121, 122. Ces exemples visent d'une part à montrer l'influence de la forme des ouvertures latérales 121, 122 et de la face supérieure 30 sur un plan de positionnement naturel pour la main de l'utilisateur et d'autre part qu'une multitude de formes d'ouverture latérale 121, 122 sont possibles

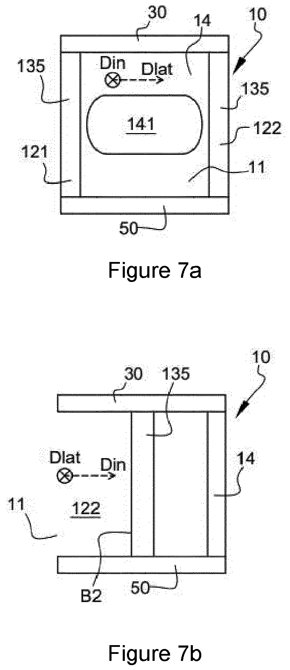

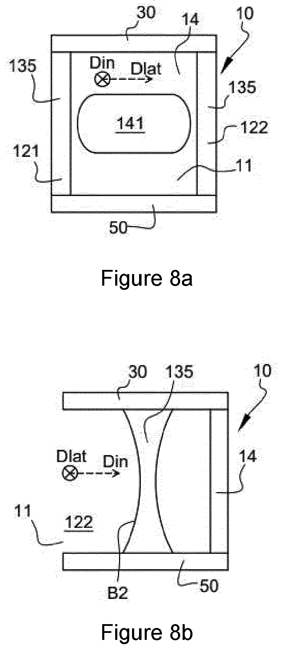

Les figures 7a, 7b, 8a, 8b illustrent un deuxième mode de réalisation d'ouverture latérale 121, 122.

Les figures 7a et 8a illustrent un compartiment utilisateur 10 d'un dispositif de capture d'empreinte biométrique 1 posé sur un sol horizontal tel que sur la figure 1, vu depuis l'ouverture frontale 11, selon la direction d'insertion Din. La paroi de fond 14 comprend un orifice de passage 141 oblong.

Les figures 7b et 8b illustrent le compartiment utilisateur 10 des figures 7a et 8a, vu depuis un côté, selon direction latérale Dlat.

A la différence du premier mode de réalisation, le compartiment utilisateur 10 ne comprend pas de deux faces latérales 131, 132.

Le compartiment utilisateur 10 comprend deux ouvertures latérales 121, 122 délimitées par deux piliers 135.

Chaque ouverture latérale 121, 122 est délimitée par un pilier 135 formant la butée de positionnement B1, B2.

Deux piliers 135 s'étendent de part et d'autre de l'ouverture frontale 11, depuis la face supérieure 30 du compartiment utilisateur 10 jusqu'au compartiment électronique 20 ou jusqu'à une base 50 du compartiment utilisateur 10 ouverte sur le compartiment électronique 20.

Selon une première variante de réalisation illustrée sur les figures 7a et 7b, chaque pilier 135 est en forme de tige droite cylindrique.

Selon une deuxième variante de réalisation illustrée sur les figures 8a et 8b, chaque pilier 135 est évasé en ses extrémités.

Ces deux variantes illustrent deux exemples non limitatifs de forme possible pour les piliers 135.

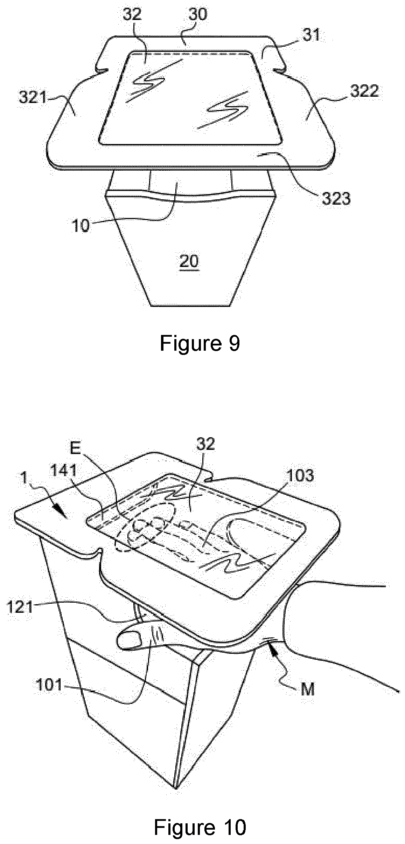

Comme représenté sur les figures 9 et 10, la face supérieure 30 comporte une portion transparente 32 permettant à l'utilisateur de voir au moins en partie sa main pour en ajuster le positionnement au sein dudit compartiment utilisateur 10.

La portion transparente 32 est apte à transmettre au moins 10% de la lumière qu'elle reçoit.

Avantageusement, la portion transparente 32 permet à l'utilisateur introduisant une partie de main au sein du compartiment utilisateur 10, de voir l'orifice de passage 141 dudit compartiment utilisateur 10.

Avantageusement, la portion transparente 32 permet à l'utilisateur introduisant une partie de main au sein du compartiment utilisateur 10, de voir la butée de positionnement B1, B2 de l'ouverture latérale 121, 122.

Le compartiment électronique 20 comprend des moyens d'éclairage aptes à générer au moins un faisceau lumineux en direction du compartiment utilisateur 10 pour éclairer une partie de main introduite dans le compartiment utilisateur 10 en vue de son acquisition d'image d'empreinte. Le compartiment électronique 20 comprend des moyens d'acquisition comprenant une caméra.

La caméra comporte un axe optique orienté de préférence perpendiculairement au plan de positionnement P pour la main

Selon un exemple de réalisation, les moyens d'éclairage sont aptes à générer plusieurs faisceaux lumineux colinéaires disposés de manière annulaire autour de la caméra.

Selon un autre exemple de réalisation, les moyens d'éclairage sont aptes à générer un unique faisceau lumineux incliné d'un angle compris entre 10° à 30° par rapport à un axe optique de la caméra.

Les moyens d'éclairage peuvent être aptes à générer une lumière diffuse, par exemple au moyen d'un diffuseur placé devant une source d'éclairage. Alternativement, les moyens d'éclairage peuvent comprendre une lame transparente dans laquelle est injectée de la lumière, la lame transparente présentant sur une face des parties diffusantes agencées de telle sorte à générer un faisceau lumineux diffus en sortie de ladite lame. Les moyens d'éclairage peuvent également comprendre des sources lumineuses de grande dimensions, par exemple des panneaux lumineux de technologie OLED.

Dans le cas d'une capture d'empreinte biométrique d'un réseau veineux de doigts ou de paume de main, les moyens d'éclairage sont avantageusement aptes à générer au moins un faisceau lumineux infrarouge et polarisé selon une direction prédéterminée, les moyens d'acquisition comportant un polariseur orthogonal à ladite direction prédéterminée.

Dans le cas d'une capture d'empreinte biométrique d'un réseau de crêtes et/ou sillons de la peau, les moyens d'éclairage sont avantageusement aptes à générer au moins un faisceau lumineux dans le spectre visible, avantageusement en vert ou bleu, et polarisé selon une direction prédéterminée, les moyens d'acquisition comportant un polariseur parallèle à ladite direction prédéterminée.

De manière préférentielle, une vitre de séparation 2 présente entre le compartiment utilisateur 10 et le compartiment électronique 20 est inclinée par rapport au plan de positionnement P moyen naturel pour la main de façon à éviter ou limiter les reflets directs dudit faisceau lumineux vers la caméra.

De même, une inclinaison de la face supérieure 30 par rapport au plan de positionnement P évite ou limite, lorsqu'elle comporte une portion transparente 32, des reflets dudit faisceau lumineux vers la caméra

Avantageusement, le compartiment utilisateur 10 comprend des moyens de blocage au moins partiel du faisceau lumineux généré par les moyens d'éclairage, au niveau de la portion transparente 32.

Par exemple, les moyens de blocage comprennent une lame à micro volets apte à filtrer la lumière provenant du au moins un faisceau lumineux, selon une direction dépendant de l'orientation desdits micro volets. Dans ce cas, pendant une utilisation du dispositif de capture d'empreinte biométrique 1, la direction dudit faisceau lumineux et une direction de regard de l'utilisateur vers le dispositif sont avantageusement différentes.

Selon un autre exemple, les moyens de blocage comprennent des moyens de sélection spectrale de l'au moins un faisceau lumineux. Par exemple, les moyens d'éclairage sont aptes à générer au moins un faisceau lumineux d'une première couleur, comme le bleu, et la face supérieure 30 comporte une portion transparente 32 teintée dans une autre couleur, comme le jaune.

Selon un autre exemple, les moyens de blocage comprennent des moyens de polarisation. Par exemple, les moyens d'éclairage sont aptes à générer au moins un faisceau lumineux polarisé linéairement, et un polariseur orthogonal est localisé contre la portion transparente 32 de la face supérieure 30. Avantageusement, les moyens d'éclairage sont aptes à générer un autre faisceau lumineux, non polarisé, visible pour l'utilisateur.

De manière générale, la portion transparente 32 de la face supérieure 30 peut comprendre une vitre teintée.

La face supérieure 30 peut comprendre une portion semi-opaque ou opaque 31, permettant de bloquer au moins 95% de la lumière la traversant ou ne laissant pas du tout passer la lumière.

Avantageusement, tel que représenté sur la figure 6d, la face supérieure 30 du compartiment utilisateur 10 est inclinée de sorte que le compartiment utilisateur est plus volumineux proche de l'ouverture frontale 11 que proche de la paroi de fond 14.

Un tel agencement est ergonomique puisqu'il permet une ouverture plus grande du compartiment utilisateur 10 destiné à recevoir une partie de main proche du poignet, plus épaisse que l'extrémité des doigts de ladite main.

De plus, lorsque la face supérieure 30 comprend une portion transparente 32, l'inclinaison de ladite face supérieure peut permettre d'éliminer ou limiter des reflets directs d'un faisceau lumineux des moyens d'éclairage vers une caméra des moyens d'acquisition.

Sur les figures, la portion transparente 32 est coplanaire avec la portion semi-opaque ou opaque 31.

Cependant, pour d'avantage limiter des reflets directs d'un faisceau lumineux des moyens d'éclairage vers une caméra des moyens d'acquisition, il peut être avantageux que la portion transparente 32 ne soit pas coplanaire avec la portion semi-opaque ou opaque 31.

Par exemple, la portion transparente 32 est inclinée par rapport au plan de positionnement P selon un premier angle de manière à limiter des reflets de lumière vers les moyens d'acquisition alors que le reste de la face supérieure qui comprend la portion semi-opaque ou opaque 31, est incliné par rapport au plan de positionnement P selon un deuxième angle différent du premier angle, de manière à donner au compartiment utilisateur 10 une forme ergonomique pour le passage d'une main en son sein, par l'ouverture frontale 11.

La portion semi-opaque ou opaque 31 peut également être parallèle au plan de positionnement P, le deuxième angle étant égal à zéro.

Avantageusement, le compartiment utilisateur 10 comprend une visière de blocage 321, 322, 323 au niveau d'au moins une ouverture parmi l'ouverture latérale 121, 122 et l'ouverture frontale 11.

Une visière de blocage 321, 322, 323 est apte à bloquer au moins une partie d'un faisceau lumineux généré par les moyens d'éclairage du compartiment électronique 20, au niveau d'une ouverture. Autrement dit, une visière de blocage 321, 322, 323 est apte à bloquer au moins une partie d'un faisceau lumineux sortant par une ouverture.

Ainsi, de la lumière venant du compartiment électronique 20 et passant par lesdites ouvertures n'éblouit pas l'utilisateur. L'effet technique de la visière de blocage 321, 322, 323 est un effet anti-éblouissement pour l'utilisateur.

Le compartiment utilisateur 10 peut également comprendre une visière de blocage au niveau de l'orifice de passage 141 (non représentée sur les figures).

Dans le cas où le compartiment utilisateur 10 ne comprend pas de paroi de fond, une visière de blocage peut être localisée au niveau de l'ouverture faisant face à l'ouverture frontale 11. Préférentiellement, la face supérieure 30 comprend ladite visière de blocage 321, 322, 323.

Sur les figures 9, 10 et 11, la face supérieure 30 s'étend au-delà des faces latérales 131, 132 du compartiment utilisateur et au-devant de l'ouverture frontale 11, les parties protubérantes formant des visières de blocage 321, 322, 323.

Sur les figures 9, 10 et 11, une visière de blocage 321, 322, 323 est faite de matière avec la portion semi-opaque ou opaque 31 de la face supérieure 30.

Sur les figures 1, 4, 5a et 5b, une visière de blocage 321, 322, 323 est faite de matière avec la portion transparente 32 de la face supérieure 30.

Qu'elle soit de matière ou non avec la portion transparente 32, dans le cas où la visière de blocage 321, 322, 323 est transparente et à l'instar des moyens de blocage de la portion transparente 32, la visière de blocage est apte à bloquer la lumière par sélection spectrale, par polarisation ou par sélection angulaire avec des micro-volets ou des filtres interférentiels.

Alternativement, une visière de blocage 321, 322, 323 peut être distincte de la face supérieure 30 et montée fixe ou mobile au compartiment utilisateur 10, par exemple à la face supérieure 30 ou à une face latérale 131, 132.

Avantageusement, tel que représenté sur les figures 9 à 11, le compartiment utilisateur 10 comprend trois visières de blocage : une première visière de blocage 321 localisée au niveau d'une première ouverture latérale 121, une deuxième visière de blocage 322 localisée au niveau d'une deuxième ouverture latérale 122 et une troisième visière de blocage 323 localisée au niveau de l'ouverture frontale 11.

Il n'est pas toujours utile que le compartiment utilisateur 10 comprenne une visière de blocage 321, 322, 323. En particulier, dans le cas où le faisceau lumineux généré par le compartiment 20 est un faisceau lumineux infrarouge ou ultraviolet, le problème d'éblouissement de l'utilisateur ne se pose pas. Ceci est par exemple applicable dans le cas pour un dispositif de capture d'empreinte biométrique d'un réseau veineux de doigts ou de paume de main.

Avantageusement, la longueur d'onde pour un faisceau infrarouge est comprise entre 700 et 2000 nm, de préférence entre 700 et 1050 nm.

Avantageusement, la longueur d'onde pour un faisceau ultraviolet est comprise entre 320 et 400 nm, de préférence entre 370 et 400 nm.

Avantageusement, la lumière extérieure au dispositif de capture d'empreinte biométrique 1 transmise par la portion transparente est bloquée par les moyens d'acquisition. Les moyens d'acquisition peuvent ainsi comprendre un filtre spectral, des micro-volets, un filtre interférentiel configuré pour réaliser une sélection angulaire, ou incorporer un polariseur.

Le compartiment électronique 20 comprend :
- des moyens d'acquisition d'une image d'une empreinte de la partie d'une main d'un utilisateur,
- des moyens d'éclairage de ladite partie d'une main.

Les moyens d'acquisition comprennent une caméra apte à acquérir une image dans la zone d'acquisition Z du compartiment utilisateur 10.

Les moyens d'acquisition peuvent comprendre une pluralité de caméras pour acquérir une pluralité d'images de plusieurs sous-zones de la zone d'acquisition Z, la pluralité de sous-zones couvrant la zone d'acquisition Z, les axes optiques des caméras étant colinéaires ou non.

Les moyens d'acquisition peuvent comprendre une pluralité de caméras pour acquérir une pluralité d'images selon plusieurs points de vue, une caméra étant par exemple apte à acquérir une image d'une paume de l'écrivain et/ou en vue d'une reconstruction d'image en trois dimensions. La paume de l'écrivain est la partie du côté de la paume qui reste normalement contre le papier lorsque l'on écrit. La paume de l'écrivain est connue dans le domaine biométrie sous le nom de « writer's palm » en anglais.

Les moyens d'éclairage sont aptes à générer au moins un faisceau lumineux en direction du compartiment utilisateur pour éclairer ladite partie de main introduite dans le compartiment utilisateur 10 en vue de l'acquisition d'une image de son empreinte.

La combinaison des moyens d'acquisition et des moyens d'éclairage permet d'obtenir des images d'empreinte de bonne qualité en vue d'une authentification de l'utilisateur ultérieure.

Avantageusement, le dispositif de capture d'empreinte biométrique 1 comprend des moyens pour informer l'utilisateur qu'il peut retirer du compartiment utilisateur 10, sa main, sa paume, son ou ses doigts, après l'acquisition d'une ou plusieurs images par les moyens d'acquisition et ainsi arrêter sa progression de sa main, sa paume ou ses doigts au sein du compartiment utilisateur selon la direction d'insertion Din de la main.

Selon un exemple d'acquisition d'une image d'empreinte biométrique des quatre doigts index, majeur, annulaire, auriculaire et de la paume d'une main, des acquisitions d'images par les moyens d'acquisition commencent au franchissement de la limite L de la zone d'acquisition Z du doigt le plus long de la main. La main progresse au sein du compartiment utilisateur du dispositif selon la direction d'insertion Din. Lorsque les acquisitions d'images sont terminées, le dispositif informe l'utilisateur qu'il peut retirer sa main.

Si la zone d'acquisition Z ne couvre pas la totalité de la partie de main dont l'empreinte doit être capturée par le dispositif, les moyens d'acquisition peuvent acquérir plusieurs images au cours de la progression de la main au sein du compartiment utilisateur 10. Ensuite les images sont assemblées pour reconstituer l'image de l'empreinte biométrique complète de ladite partie de main.

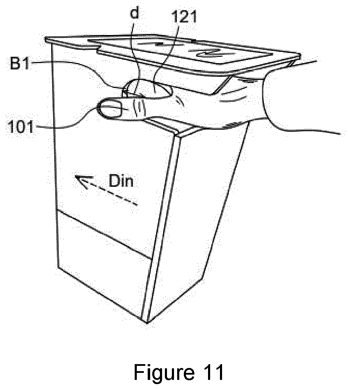

Un placement correct pour l'acquisition d'une empreinte biométrique de la paume de la main correspond par exemple à distance entre ladite main et la butée de positionnement B1, B2 inférieure ou égale à une distance prédéterminée d tel que représenté en figure 2 et figure 11.

La distance entre la main et la butée de positionnement B1, B2 peut être déterminée par un capteur optique tel qu'un télémètre ou une caméra, ou par la détection d'une coupure d'un faisceau infrarouge localisé à proximité de la butée de positionnement B1, B2.

Une caméra des moyens d'acquisition 20 peut par exemple être utilisée pour la détermination de ladite distance entre la main et de la butée de positionnement B1, B2.

Les moyens pour informer l'utilisateur qu'il peut retirer sa main comprennent par exemple des moyens d'envoi d'un signal sonore, d'affichage d'un message ou d'un pictogramme, ou encore d'allumage d'un témoin lumineux.

Le positionnement latéral de la main, selon la direction latérale Dlat, au sein du compartiment utilisateur 10 n'a pas besoin d'être contrôlé puisque la butée de positionnement B1, B2 et/ou la face latérale 131, 132 contraint la partie de la main au sein du compartiment utilisateur 10 à rester dans la zone d'acquisition Z, en particulier dans le cas où le pouce de la main est positionné dans une ouverture latérale 121, 122, lors d'une acquisition de la paume d'une main ou des quatre doigts index, majeur, annulaire et auriculaire de la main.

Les figures 10 et 11 illustrent une main M d'un utilisateur au sein d'un dispositif de capture d'empreinte biométrique selon l'invention. Le pouce 101 de l'utilisateur est écarté des autres doigts 103 de la main.

Comme visible sur la figure 10, l'utilisateur peut voir en partie sa main, au travers de la portion transparente 32 de la face supérieure 30 du compartiment utilisateur 10. L'extrémité E de ladite main de l'utilisateur est visible par l'utilisateur, ainsi qu'une partie de l'orifice de passage 141. Le pouce 101 est localisé dans l'ouverture latérale 121 du compartiment utilisateur.

Comme visible sur la figure 11, la main de l'utilisateur a été introduite selon une direction d'insertion Din et est localisée dans le compartiment utilisateur. Le pouce 101 est localisé à une distance d non nulle de la butée de positionnement B1 de l'ouverture latérale 121 du compartiment utilisateur.

On remarque que l'utilisateur est incité à écarter le pouce 101 du reste des doigts 103 de sa main M, sinon il serait gêné par la butée de positionnement B1 pour introduire complètement sa main au sein du compartiment utilisateur.

On remarque également que l'utilisateur ne peut pas introduire sa main très loin selon la direction d'insertion Din au sein du compartiment utilisateur car il est gêné par la butée de positionnement B1 qui va bloquer la progression de sa main au sein dudit compartiment utilisateur.

Ainsi, la butée de positionnement B1 incite l'utilisateur à écarter le pouce 101 du reste des doigts de sa main M et permet un bon positionnement au sein du compartiment utilisateur 10, ledit positionnement pouvant être contrôlé grâce à la portion transparente 32 de la face supérieure 30 du compartiment utilisateur 10 qui lui permet de voir au moins en partie, sa main au sein dudit compartiment utilisateur 10.

Optionnellement, le dispositif de l'invention peut comprendre un écran localisé par exemple au-dessus du compartiment utilisateur, pour guider l'utilisateur sur des placements de main successifs à effectuer au sein du compartiment utilisateur.

Le dispositif de l'invention est particulièrement adapté à la capture d'empreinte biométrique de la paume d'une main.

Le dispositif de l'invention peut également être utilisé pour la capture d'empreinte biométrique des deux pouces des deux mains d'un utilisateur, les ouvertures latérales 121, 122 permettant le passage des pouces au sein du compartiment utilisateur 10, les autres doigts étant positionnés à l'extérieur du compartiment utilisateur 10, les paumes des deux mains se faisant face.

L'ouverture latérale 11 permet le passage d'un ou plusieurs doigts pour la capture d'empreinte de doigts, tel que connu de l'état de la technique.

## Revendications

1. Dispositif de capture d'empreinte biométrique (1) sans contact d'une partie d'une main d'un utilisateur, apte à acquérir une partie d'une main étant une partie d'une paume de main, comprenant un compartiment électronique (20) et un compartiment utilisateur (10), le compartiment utilisateur (10) comportant une ouverture frontale (11) adaptée pour le passage de la paume d'une main au sein dudit compartiment utilisateur (10), le compartiment électronique (20) comprenant des moyens d'acquisition d'une image d'une empreinte de ladite partie d'une main, le compartiment utilisateur (10) comprenant au moins une ouverture latérale (121, 122) permettant le passage du pouce de la main de l'utilisateur, **caractérisé en ce que** l'ouverture latérale (121, 122) s'étend jusqu'à l'ouverture frontale (11) pour former un espace ouvert continu, l'ouverture latérale (121, 122) étant délimitée selon une direction d'insertion (Din) de la main de l'utilisateur dans le compartiment utilisateur (10) par une butée de positionnement (B1, B2) de la main de l'utilisateur, de sorte à inciter une introduction de la paume d'une main, pouce écarté, au sein du compartiment utilisateur (10), la butée de positionnement gênant une introduction complète de la main.

2. Dispositif de capture d'empreinte biométrique (1) selon la revendication précédente, le compartiment utilisateur (10) comprenant une zone d'acquisition (Z) délimitée du côté de l'ouverture frontale (11) par une limite (L), la butée de positionnement (B1, B2) étant localisée à une distance à la butée (dB) comprise entre cinquante et quatre-vingts millimètres de ladite limite (L).

3. Dispositif de capture d'empreinte biométrique (1) selon l'une quelconque des revendications précédentes, le compartiment utilisateur (10) comprenant un orifice de passage (141) apte à laisser dépasser dudit compartiment utilisateur (10) une extrémité de ladite main de l'utilisateur, l'orifice de passage (141) faisant face à l'ouverture frontale.

4. Dispositif de capture d'empreinte biométrique selon l'une quelconque des revendications précédentes, le compartiment utilisateur (10) comprenant une face supérieure (30) comportant une portion transparente (32) permettant à l'utilisateur de voir au moins une portion de la partie de main pour en ajuster le positionnement au sein dudit compartiment utilisateur (10).

5. Dispositif de capture d'empreinte biométrique selon la revendication précédente, le compartiment électronique (20) comprenant des moyens d'éclairage aptes à générer au moins un faisceau lumineux en direction du compartiment utilisateur (10), le compartiment utilisateur (10) comprenant des moyens de blocage au moins partiel d'un faisceau lumineux provenant du compartiment électronique (20) au niveau de la portion transparente (32).

6. Dispositif de capture d'empreinte biométrique selon l'une quelconque des revendications précédentes, le compartiment utilisateur (10) comprenant une visière de blocage (321, 322, 323) apte à bloquer un faisceau lumineux provenant du compartiment électronique (20), au niveau d'au moins une ouverture parmi l'ouverture latérale (121, 122) et l'ouverture frontale (11).

7. Dispositif de capture d'empreinte biométrique selon l'une quelconque des revendications précédentes, le compartiment utilisateur (10) comprenant une face latérale (131, 132) comportant ladite ouverture latérale (121, 122) s'étendant depuis l'ouverture frontale (11) du compartiment utilisateur jusqu'à la butée de positionnement (B1, B2).

8. Dispositif de capture d'empreinte biométrique selon la revendication précédente, l'ouverture latérale (11) étant délimitée par la butée de positionnement (B1, B2), un bord supérieur (BT1, BT2) et un bord inférieur (BB1, BB2), au moins un des bords (BT1, BT2, BB1, BB2) parmi le bord supérieur (BT1, BT2) et le bord inférieur (BB1, BB2) étant agencé de sorte à définir un plan de positionnement (P) moyen naturel pour la main sensiblement parallèle à l'au moins un des bords ou équidistant du bord supérieur (BT1, BT2) et du bord inférieur (BB1, BB2).

9. Dispositif de capture d'empreinte biométrique selon l'une quelconque des revendications précédentes, comprenant deux ouvertures latérales (121, 122) de part et d'autre de l'ouverture frontale (11), une première ouverture latérale (121) permettant le passage du pouce de la main droite de l'utilisateur, une deuxième ouverture latérale (122) permettant le passage du pouce de la main gauche de l'utilisateur.

10. Dispositif de capture d'empreinte biométrique (1) selon l'une quelconque des revendications précédentes, comprenant des moyens pour informer un utilisateur d'une fin de session d'acquisition par les moyens d'acquisition, en fonction d'une distance entre ladite partie de main et la butée de positionnement (B1, B2).
